(19) Europäisches Patentamt
European Patent Office
Office européen des brevets

(11) Veröffentlichungsnummer : **0 440 960 B1**

# (12) EUROPÄISCHE PATENTSCHRIFT

(45) Veröffentlichungstag der Patentschrift : **23.02.94 Patentblatt 94/08**

(51) Int. Cl.$^5$ : **C07D 229/00**

(21) Anmeldenummer : **90124850.0**

(22) Anmeldetag : **20.12.90**

## (54) Verfahren zur Verbesserung der Fliessfähigkeit von dimerisiertem 2,4-Toluylendiisocyanat.

(30) Priorität : **18.01.90 DE 4001247**

(43) Veröffentlichungstag der Anmeldung : **14.08.91 Patentblatt 91/33**

(45) Bekanntmachung des Hinweises auf die Patenterteilung : **23.02.94 Patentblatt 94/08**

(84) Benannte Vertragsstaaten : **AT BE CH DE ES FR GB IT LI NL**

(56) Entgegenhaltungen : **EP-A- 0 308 710**

(73) Patentinhaber : **BAYER AG**
**D-51368 Leverkusen (DE)**

(72) Erfinder : **Kopp, Richard, Dr.**
**Bilharzstrasse 15**
**W-5000 Köln 80 (DE)**
Erfinder : **Grögler, Gerhard, Dr.**
**von-Diergardt-Strasse 48**
**W-5090 Leverkusen 1 (DE)**
Erfinder : **Hess, Heinrich, Dr.**
**Auf der Schildwache 13a**
**W-5000 Köln 80 (DE)**
Erfinder : **Georgias, Lutz, Dr.**
**Friedrich-Ebert-Strasse 5**
**W-4047 Dormagen (DE)**
Erfinder : **Hurnik, Helmut, Dipl.-Ing.**
**Dechant-Krey-Strasse 24**
**W-5090 Leverkusen 3 (DE)**
Erfinder : **Thomas, Hans Dieter**
**Am Katterbach 66**
**W-5060 Bergisch Gladbach 2 (DE)**

EP 0 440 960 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Verbesserung der Fließfähigkeit und zur Verhinderung der Agglomeration von feingemahlenem dimerisiertem 2,4-Toluylendiisocyanat durch Zusatz von Fällungs- und/oder pyrogenen Kieselsäuren mit hydrophober Oberfläche.

Feingemahlenes dimerisiertes 2,4-Toluylendiisocyanat (Formel (I), Handelsprodukt Desmodur® TT, Fa. Bayer AG) ist das zur Zeit einzige im Handel erhältliche

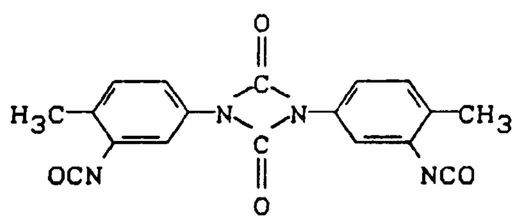

(I)

feste, höherschmelzende Diisocyanat. Es wird hauptsächlich in PVC-Haftvermittlern und bei der Herstellung von Polyurethankautschuk eingesetzt, wo es zum Erreichen eines homogenen Endproduktes in möglichst feiner Form verwendet wird. Ein neueres Einsatzgebiet sind Polyurethan-Einkomponentensysteme auf der Basis von festen Polyisocyanaten.

Das Handelsprodukt Desmodur® TT liegt in Form eines weißen Pulvers mit einer Teilchengröße von 2 bis 25 µm vor. Beim Transport, bei der Lagerung und insbesondere bei Anwendungen, bei denen das Desmodur® TT-Pulver in viskosen Flüssigkeiten dispergiert werden muß, kann die Neigung der feinen Feststoffpartikel zur Verdichtung und Agglomeration Schwierigkeiten bereiten.

Aufgabe war es daher, die Fließfähigkeit des feinteiligen, dimerisierten 2,4-Toluylendiisocyanates zu verbessern bzw. die Agglomerationsneigung des Feststoffes zu verringern.

Diese Aufgabe konnte gelöst werden, indem dem Polyisocyanat bestimmte Kieselsäuretypen als Additive zugesetzt werden.

Gegenstand der Erfindung ist somit ein Verfahren zur Verbesserung der Fließfähigkeit und zur Verhinderung der Agglomeration von feinteiligem, dimerisiertem 2,4-Toluylendiisocyanat durch Zusatz von Fällungs- und/oder pyrogenen Kieselsäuren mit hydrophober Oberfläche.

Die Fällungs- und/oder pyrogenen Kieselsäuren mit hydrophober Oberfläche werden vorzugsweise in Mengen von 0,01 bis 10 Gew.-% zugesetzt, besonders bevorzugt in Mengen von 0,1 bis 2,5 Gew.-%.

Diese Kieselsäuren können zu einem beliebigen Zeitpunkt des Herstellungsprozesses von dimerisiertem 2,4-Toluylendiisocyanat zugesetzt werden.

Bevorzugt werden die Fällungs- und/oder pyrogenen Kieselsäuren mit hydrophober Oberfläche dem Reaktionsgemisch nach Ausfällung des festen, dimerisierten 2,4-Toluylendiisocyanates und vor dessen Aufarbeitung und Mahlung zugesetzt.

In einer weiteren Verfahrensvariante werden die Kieselsäuren dem bereits getrockneten und gemahlenen Polyisocyanat zugesetzt und mit diesem intensiv vermischt.

Außer den Fällungs- und/oder pyrogenen Kieselsäuren mit hydrophober Oberfläche können auch weitere Additive zugesetzt werden. Weitere Additive sind z.B. $CaCO_3$, $BaSO_4$, Talkum, Kieselsäuren mit hydrophiler Oberfläche, $Al_2O_3$ oder $Sb_2O_3$ und dergleichen.

Ein weiterer Gegenstand der Erfindung ist feinteiliges, dimerisiertes 2,4-Toluylendiisocyanat mit verbesserter Fließfähigkeit, wobei das Produkt eine mittlere Teilchengröße von 2 bis 25 µm und 0,01 bis 10 Gew.-% Fällungs-und/oder pyrogene Kieselsäuren mit hydrophober Oberfläche aufweist.

Das erfindungsgemäße feinteilige, dimerisierte 2,4-Toluylendiisocyanat wird bei der Herstellung von Urethangruppen und/oder Harnstoffgruppen enthaltenden Polymeren als Isocyanatkomponente verwendet.

Fällungs- und pyrogene Kieselsäuren mit hydrophober Oberfläche sind seit langem bekannt und im Handel erhältlich. Genannt seien z.B. die Produkte Aerosil R 202, Aerosil R 805, Aerosil R 812, Aerosil R 972, Aerosil R 974, Aerosil R 976, Sipernat D 10, Sipernat D 17, die von der Fa. Degussa AG angeboten werden. Kieselsäuretypen mit hydrophober Oberfläche, die von anderen Herstellern angeboten werden, sind ebenfalls geeignet.

Der Zusatz dieser Kieselsäuren kann zum bereits aufgearbeiteten und getrockneten Polyisocyanat erfolgen. Es ist aber auch möglich, die Kieselsäuren zu einem oder mehreren beliebigen Zeitpunkten der Herstellung des dimerisierten 2,4-Toluylendiisocyanates, gegebenenfalls aufgeteilt auf mehrere Portionen, zuzusetzen, wie z.B. nach der Ausfällung, vor der Abtrennung, vor der Trocknung, vor der Mahlung oder nach der Mahlung des Feststoffes. Die Kieselsäuren können auch als Suspension in einem geeigneten Lösungsmittel eingesetzt werden.

Methoden zur Handhabung der Kieselsäuren werden z.B. in der Druckschrift "Schriftenreihe Pigmente, Nummer 28", Fa. Degussa AG genannt.

Die Erfindung soll anhand der nachfolgenden Beispiele näher erläutert werden.

Beispiele

Für die Durchführung der Versuche wurde käufliches, feingemahlenes, dimerisiertes 2,4-Toluylendiisocyanat (Teilchengröße von 2 bis 25 µm) eingesetzt (Desmodur® TT der Fa. Bayer AG).

Zur Herstellung der Proben wurden jeweils nacheinander in eine neue, unbenutzte 500 ml Glasflasche 100 Gew.-Teile Desmodur® TT, die jeweilige Menge Kieselsäure und nochmals 100 Gew.-Teile Desmodur® TT eingefüllt. Dann wurde die verschlossene Glasflasche 1 Minute intensiv durchgeschüttelt.

An diesen Proben wurden folgende Messungen durchgeführt:

1. Schüttkegelversuch

Über einem Metallvollzylinder mit einem Durchmesser von 50 mm und einer Höhe von ca. 80 mm wird in einem Abstand von 3 bis 10 cm ein Metallsieb befestigt. Auf das Sieb wird das zu prüfende Pulver geschüttelt und mit Hilfe eines Pinsels von Hand langsam durch das Sieb gedrückt.

Das herabfallende Pulver baut auf dem Metallzylinder einen Schüttkegel auf, dessen Höhe bzw. Böschungswinkel gemessen wird. Diese Größen sind ein direktes Maß für die Rieselfähigkeit des geprüften Pulvers. Je niedriger der Schüttkegel umso besser ist die Rieselfähigkeit bzw. Fließfähigkeit des Pulvers.

2. Auslaufversuch

Für die Messungen werden mit einer Siliconemulsion hydrophobierte, sanduhrähnliche Auslaufgefäße mit definierten, unterschiedlichen Auslaufweiten (13 mm, 12 mm, 9 mm, 8 mm, 5 mm) eingesetzt.

Das zu prüfende Pulver wird in das Meßgefäß eingefüllt, wobei man die Auslauföffnung anfangs verschlossen hält. Dann öffnet man die Auslauföffnung und mißt die zum Ausfließen des Pulvers benötigte Zeit und die aus dem Meßgefäß ausgelaufene Pulvermenge im Verhältnis zur gesamten, in das Meßgefäß gegebenen Menge.

Man beginnt die Messungen mit dem Meßgefäß mit der größten Auslaufweite und geht dann Schritt für Schritt zu den Gefäßen mit kleinerer Auslaufweite über. Je kleiner die Auslaufweite bei noch auslaufendem Pulver ist, umso besser ist das Fließvermögen des Pulvers.

Beispiel 1

In einer Versuchsreihe wurden eine unmodifizierte Probe Desmodur® TT und mehrere, mit 1 Gew.-% verschiedener erfindungsgemäßer Kieselsäuren mit hydrophober Oberfläche versetzte Proben dem Schüttkegeltest und dem Auslaufversuch mit Auslaufweite 13 mm unterzogen. Tabelle 1 zeigt die gefundenen Ergebnisse.

Tabelle 1

| Additiv | Kegelhöhe (mm) | Trichterauslauf- zeit (s) | Trichterauslauf- menge (%) |
|---|---|---|---|
| ohne | 68 | ∞ | 0 |
| Aerosil R 202[1)] | 44 | 3 | 70 |
| Aerosil R 805[1)] | 43 | 1 | 100 |
| Aerosil R 812[1)] | 28 | 1 | 100 |
| Aerosil R 972[1)] | 41 | 1 | 100 |
| Aerosil R 974[1)] | 36 | 1 | 100 |
| Sipernat D 10[2)] | 47 | 1 | 100 |
| Sipernat D 17[2)] | 34 | 1 | 100 |

1) Handelsprodukte der Firma Degussa AG physikalisch chemische Daten der Kieselsäuren siehe Druckschrift: "Schriftenreihe Pigmente, Nr. 23, Aerosil als Verdickungsmittel für Flüssigkeiten", Firma Degussa AG;

2) Handelsprodukte der Firma Degussa AG physikalisch chemische Daten der Kieselsäuren siehe "Fällungskieselsäuren und Silikate, Herstellung, Eigenschaften und Anwendungen", Fa. Degussa AG

Wie deutlich zu erkennen ist, wurden bei Einsatz der erfindungsgemäßen Kieselsäuren die Kegelhöhen im Vergleich zur Kegelhöhe des Desmodur® TT ohne Kieselsäuren wesentlich verringert und ein Auslaufen des Pulvers aus dem Meßgefäß überhaupt erst ermöglicht.

Beispiel 2

Mit den Additiven Aerosil R 812, Aerosil R 805, Aerosil R 974 und Sipernat D 17 wurden Auslaufversuche wie in Beispiel 1 durchgeführt, nur wurden die Auslaufzeiten und -mengen in Meßgefäßen mit kleineren Auslaufweiten gemessen und die Additivmengen variiert. Tabelle 2 zeigt die Ergebnisse.

Tabelle 2

| Additiv | Additivmenge (Gew.-%) | Auslaufzeit (s) / Auslaufmenge (%) Trichtergrößen 13 mm | 12 mm | 9 mm | 8 mm | 5 mm |
|---|---|---|---|---|---|---|
| Aerosil R 812 | 0,25 | 3,0/80 | * | * | * | * |
| Aerosil R 812 | 0,50 | 1,0/100 | 1,0/100 | 2,0/90 | * | * |
| Aerosil R 812 | 1,00 | 1,0/100 | 1,0/100 | 1,5/100 | 1,5/100 | 5,0/90 |
| Aerosil R 812 | 1,50 | 1,0/100 | 1,0/100 | 1,0/100 | 1,5/100 | 6,0/90 |
| Aerosil R 812 | 2,00 | 1,0/100 | 1,0/100 | 1,0/100 | 2,0/100 | 4,0/90 |
| Aerosil R 805 | 0,25 | 1,0/30 | * | * | * | * |
| Aerosil R 805 | 0,50 | 1,0/80 | * | * | * | * |
| Aerosil R 805 | 1,00 | 1,0/100 | 1,0/100 | 3,0/70 | * | * |
| Aerosil R 805 | 1,50 | <1/100 | 1,0/100 | 2,5/100 | 3,0/90 | * |
| Aerosil R 805 | 2,00 | <1/100 | <1/100 | 2,5/100 | 2,5/90 | * |
| Aerosil R 974 | 0,25 | 1,5/50 | * | * | * | * |
| Aersoil R 974 | 0,50 | 1,5/100 | 1,5/100 | 2,0/50 | * | * |
| Aerosil R 974 | 1,00 | 1,0/100 | 1,0/100 | 3,0/100 | 2,5/100 | 5,0/80 |
| Aerosil R 974 | 1,50 | 1,0/100 | 1,0/100 | 2,5/100 | 3,0/100 | 5,0/90 |
| Aerosil R 974 | 2,00 | <1/100 | <1/100 | 2,5/100 | 2,5/100 | 5,0/90 |
| Sipernat D 17 | 0,25 | 1,5/70 | * | * | * | * |
| Sipernat D 17 | 0,50 | 1,5/100 | 1,5/100 | 2,0/100 | 3,0/100 | 4,5/80 |
| Sipernat D 17 | 1,00 | 1,0/100 | 1,5/100 | 2,0/100 | 3,0/100 | 4,5/80 |
| Sipernat D 17 | 1,50 | <1/100 | 1,0/100 | 2,0/100 | 2,5/100 | 4,0/90 |
| Sipernat D 17 | 2,00 | <1/100 | 1,0/100 | 2,0/100 | 2,5/100 | 4,0/90 |

* bedeutet ∞ sec/0%
∞ unendlich

Die Testergebnisse mit den Auslaufgefäßen mit kleineren Auslaufweiten bestätigen die im vorherigen Beispiel gefundenen Resultate, ermöglichen aber eine bessere Differenzierung der positiven Wirkung auf das Fließvermögen des Desmodur® TT zwischen den einzelnen Kieselsäuren.

Beispiel 3 (Vergleichsbeispiel)

Unter den gleichen Bedingungen wie in Beispiel 1 wurden zum Vergleich Kieselsäuren, die keine hydrophobe Oberfläche aufweisen, oder andere Additive eingesetzt. Wie Tabelle 3 zeigt, wird bei diesen Kieselsäuren praktisch keine Verbesserung des Fließverhaltens beobachtet.

Tabelle 3

| Additiv | Kegelhöhe (mm) | Trichterauslaufzeit (S) | Trichterauslaufmenge (%) |
|---|---|---|---|
| ohne | 68 mm | ∞ | 0 % |
| Aerosil 130[1)] | 55 mm | 2 sec | 90 % |
| Aerosil 200[1)] | 57 mm | ∞ | 0 % |
| Aerosil 380[1)] | 57 mm | 4 sec | 80 % |
| Aerosil OX 50[1)] | 61 mm | 3 sec | 70 % |
| Sipernat 22[2)] | 59 mm | 1 sec | 80 % |
| Sipernat 22 LS[2)] | 60 mm | 1 sec | 70 % |
| Sipernat 44[2)] | 58 mm | ∞ | 0 % |
| FK 320[2)] | 48 mm | 2 sec | 60 % |
| FK 320 DS[2)] | 47 mm | 1 sec | 80 % |
| Durosil[2)] | 51 mm | <1 sec | 50 % |
| Wessalon S[2)] | 54 mm | 1 sec | 70 % |
| Extrusil[2)] | 57 mm | 1 sec | 80 % |
| Transpafill[2)] | 48 mm | 1 sec | 60 % |
| Vulkasil S[4)] | 60 mm | 1 sec | 30 % |
| Vulkasil N[4)] | 58 mm | 1 sec | 80 % |
| Talkum rein | 58 mm | 1 sec | 50 % |
| Aluminium-oxid C[3)] | 54 mm | 1 sec | 70 % |

3) Handelsprodukte der Firma Degussa AG physikalisch chemische Daten siehe "Schriftenreihe Pigmente, Nr. 13, Synthetische Kieselsäuren als Hilfsmittel für die Kunststoffindustrie", Firma Degussa

4) Handelsprodukte der Firma Bayer AG physikalisch chemische Daten siehe "Anorganische Pigmente, Füllstoffe und Aktivatoren für die Gummi-Industrie", Firma Bayer AG

## Patentansprüche

### Patentansprüche für folgende Vertragsstaaten : AT, BE, CH, DE, FR, GB, IT, LI, NL

1. Verfahren zur Verbesserung der Fließfähigkeit und der Verhinderung der Agglomeration von feinteiligem, dimerisiertem 2,4-Toluylendiisocyanat, dadurch gekennzeichnet, daß dem Produkt 0,01 bis 10 Gew.-% Fällungs- und/oder pyrogene Kieselsäuren mit hydrophober Oberfläche zugesetzt werden.

2. Verfahren nach Anspruch 1, dadurch gekennzeichnet, daß 0,1 bis 2,5 Gew.-% Fällungs- und/oder pyrogene Kieselsäuren mit hydrophober Oberfläche eingesetzt werden.

3. Verfahren nach Anspruch 1 oder 2, dadurch gekennzeichnet, daß die Kieselsäuren zu einem beliebigen Zeitpunkt des Herstellungsverfahrens des dimerisierten 2,4-Toluylendiisocyanates zugesetzt werden.

4. Verfahren nach Anspruch 1 oder 2, dadurch gekennzeichnet, dar die Kieselsäuren dem Reaktionsgemisch nach Ausfällung des festen, dimerisierten 2,4-Toluylendiisocyanates und vor dessen Aufarbeitung und Mahlung zugesetzt werden.

5. Verfahren nach Anspruch 1 oder 2, dadurch gekennzeichnet, daß die Kieselsäuren dem bereits getrockneten und gemahlenen Polyisocyanat zugesetzt und mit diesem intensiv vermischt werden.

6. Verfahren nach einem der Ansprüche 1 bis 5, dadurch gekennzeichnet, daß die Kieselsäuren zusammen mit weiteren Additiven eingesetzt werden.

7. Feinteiliges, dimerisiertes 2,4-Toluylendiisocyanat mit verbesserter Fließfähigkeit, dadurch gekennzeichnet, daß das Produkt eine mittlere Teilchengröße von 2 bis 25 µm und 0,01 bis 10 Gew.-% Fällungs- und/oder pyrogene Kieselsäuren mit hydrophober Oberfläche aufweist.

8. Verwendung des feinteiligen, dimerisierten 2,4-Toluylendiisocyanates gemäß einem der Ansprüche 1 bis 7 bei der Herstellung von Urethangruppen und/oder Harnstoffgruppen enthaltenden Polymeren als Isocyanatkomponente.

**Patentansprüche für folgenden Vertragsstaat : ES**

1. Verfahren zur Verbesserung der Fließfähigkeit und der Verhinderung der Agglomeration von feinteiligem, dimerisiertem 2,4-Toluylendiisocyanat, dadurch gekennzeichnet, daß dem Produkt 0,01 bis 10 Gew.-% Fällungs- und/oder pyrogene Kieselsäuren mit hydrophober Oberfläche zugesetzt werden.

2. Verfahren nach Anspruch 1, dadurch gekennzeichnet, daß 0,1 bis 2,5 Gew.-% Fällungs- und/oder pyrogene Kieselsäuren mit hydrophober Oberfläche eingesetzt werden.

3. Verfahren nach Anspruch 1 oder 2, dadurch gekennzeichnet, daß die Kieselsäuren zu einem beliebigen Zeitpunkt des Herstellungsverfahrens des dimerisierten 2,4-Toluylendiisocyanates zugesetzt werden,

4. Verfahren nach Anspruch 1 oder 2, dadurch gekennzeichnet, daß die Kieselsäuren dem Reaktionsgemisch nach Ausfällung des festen, dimerisierten 2,4-Toluylendiisocyanates und vor dessen Aufarbeitung und Mahlung zugesetzt werden.

5. Verfahren nach Anspruch 1 oder 2, dadurch gekennzeichnet, daß die Kieselsäuren dem bereits getrockneten und gemahlenen Polyisocyanat zugesetzt und mit diesem intensiv vermischt werden.

6. Verfahren nach einem der Ansprüche 1 bis 5, dadurch gekennzeichnet, daß die Kieselsäuren zusammen mit weiteren Additiven eingesetzt werden.

7. Verwendung des feinteiligen, dimerisierten 2,4-Toluylendiisocyanates gemäß einem der Ansprüche 1 bis 6 bei der Herstellung von Urethangruppen und/oder Harnstoffgruppen enthaltenden Polymeren als Isocyanatkomponente.

**Claims**

**Claims for the following Contracting States : AT, BE, CH, DE, FR, GB, IT, LI, NL**

1. Process to improve the flowability and to prevent agglomeration of finely divided, dimerised tolylene 2,4-diisocyanate, characterised in that 0.01 to 10 wt.% of precipitated and/or pyrogenic silicas with a hydrophobic surface are added to the product.

2. Process according to claim 1, characterised in that 0.1 to 2.5 wt.% of precipitated and/or pyrogenic silicas

with a hydrophobic surface are used.

3. Process according to claim 1 or 2, characterised in that the silicas are added at any desired point during the production process of the dimerised tolylene 2,4-diisocyanate.

4. Process according to claim 1 or 2, characterised in that the silicas are added to the reaction mixture after precipitation of the solid, dimerised tolylene 2,4-diisocyanate and before it is further processed and ground.

5. Process according to claim 1 or 2, characterised in that the silicas are added to the already dried and ground polyisocyanate and are intensively mixed with it.

6. Process according to one of claims 1 to 5, characterised in that the silicas are used together with further additives.

7. Finely divided, dimerised tolylene 2,4-diisocyanate with improved flowability, characterised in that the product has an average particle size of 2 to 25 μm and 0.01 to 10 wt.% of precipitated and/or pyrogenic silicas with a hydrophobic surface.

8. Use of the finely divided, dimerised tolylene 2,4-diisocyanate according to one of claims 1 to 7 as the isocyanate component in the production of polymers containing urethane groups and/or urea groups.

**Claims for the following Contracting State : ES**

1. Process to improve the flowability and to prevent agglomeration of finely divided, dimerised tolylene 2,4-diisocyanate, characterised in that 0.01 to 10 wt.% of precipitated and/or pyrogenic silicas with a hydrophobic surface are added to the product.

2. Process according to claim 1, characterised in that 0.1 to 2.5 wt.% of precipitated and/or pyrogenic silicas with a hydrophobic surface are used.

3. Process according to claim 1 or 2, characterised in that the silicas are added at any desired point during the production process of the dimerised tolylene 2,4-diisocyanate.

4. Process according to claim 1 or 2, characterised in that the silicas are added to the reaction mixture after precipitation of the solid, dimerised tolylene 2,4-diisocyanate and before it is further processed and ground.

5. Process according to claim 1 or 2, characterised in that the silicas are added to the already dried and ground polyisocyanate and are intensively mixed with it.

6. Process according to one of claims 1 to 5, characterised in that the silicas are used together with further additives.

7. Use of the finely divided, dimerised tolylene 2,4-diisocyanate according to one of claims 1 to 6 as the isocyanate component in the production of polymers containing urethane groups and/or urea groups.

## Revendications

**Revendications pour les Etats contractants suivants : AT, BE, CH, DE, FR, GR, IT, LI, NL**

1. Procédé pour améliorer l'aptitude à l'écoulement et pour empêcher l'agglomération de 2,4-diisocyanatotoluène dimérisé finement divisé, caractérisé en ce qu'on ajoute au produit 0,01 à 10 % en poids de silices produites par précipitation et/ou par pyrogénation, à surface hydrophobe.

2. Procédé suivant la revendication 1, caractérisé en ce qu'on utilise 0,1 à 2,5 % en poids de silices produites par précipitation et/ou par pyrogénation à surface hydrophobe.

3. Procédé suivant la revendication 1 ou 2, caractérisé en ce que les silices sont ajoutées à un instant quelconque du procédé de production du 2,4-diisocyanatotoluène dimérisé.

4. Procédé suivant la revendication 1 ou 2, caractérisé en ce que les silices sont ajoutées au mélange réactionnel après la précipitation du 2,4-diisocyanatotoluène dimérisé solide et avant son traitement final et son broyage.

5. Procédé suivant la revendication 1 ou 2, caractérisé en ce que les silices sont ajoutées au polyisocyanate déjà séché et broyé et sont mélangées énergiquement avec lui.

6. Procédé suivant l'une des revendications 1 à 5, caractérisé en ce que les silices sont utilisées conjointement avec d'autres additifs.

7. 2,4-diisocyanatotoluène dimérisé finement divisé, dont l'aptitude à l'écoulement est améliorée, caractérisé en ce que le produit présente un diamètre moyen des particules de 2 à 25 µm et 0,01 à 10 % en poids de silices obtenues par précipitation et/ou par pyrogénation, à surface hydrophobe.

8. Utilisation du 2,4-diisocyanatotoluène dimérisé finement divisé suivant l'une des revendications 1 à 7 comme composant isocyanate dans la production de polymères contenant des groupes uréthanne et/ou des groupes urée.

**Revendications pour l'Etat contractant suivant : ES**

1. Procédé pour améliorer l'aptitude à l'écoulement et pour empêcher l'agglomération de 2,4-diisocyanatotoluène dimérisé finement divisé, caractérisé en ce qu'on ajoute au produit 0,01 à 10 % en poids de silices produites par précipitation et/ou par pyrogénation, à surface hydrophobe.

2. Procédé suivant la revendication 1, caractérisé en ce qu'on utilise 0,1 à 2,5 % en poids de silices produites par précipitation et/ou par pyrogénation à surface hydrophobe.

3. Procédé suivant la revendication 1 ou 2, caractérisé en ce que les silices sont ajoutées à un instant quelconque du procédé de production du 2,4-diisocyanatotoluène dimérisé.

4. Procédé suivant la revendication 1 ou 2, caractérisé en ce que les silices sont ajoutées au mélange réactionnel après la précipitation du 2,4-diisocyanatotoluène dimérisé solide et avant son traitement final et son broyage.

5. Procédé suivant la revendication 1 ou 2, caractérisé en ce que les silices sont ajoutées au polyisocyanate déjà séché et broyé et sont mélangées énergiquement avec lui.

6. Procédé suivant l'une des revendications 1 à 5, caractérisé en ce que les silices sont utilisées conjointement avec d'autres additifs.

7. Utilisation du 2,4-diisocyanatotoluène dimérisé finement divisé suivant l'une des revendications 1 à 6 comme composant isocyanate dans la production de polymères contenant des groupes uréthanne et/ou des groupes urée.